(19) Europäisches Patentamt
European Patent Office
Office européen des brevets

(11) **EP 3 514 538 B1**

(12) **EUROPEAN PATENT SPECIFICATION**

(45) Date of publication and mention
of the grant of the patent:
**04.08.2021 Bulletin 2021/31**

(51) Int Cl.:
**G01N 33/50** [(2006.01)]

(21) Application number: **19152427.1**

(22) Date of filing: **18.01.2019**

(54) **COMPOSITION FOR CONTROLLING SECRETION OF PROSTASOMES AND USE THEREOF**

ZUSAMMENSETZUNG ZUR STEUERUNG DER SEKRETION VON PROSTASOMEN UND VERWENDUNG DAVON

COMPOSITION DE RÉGULATION DE LA SÉCRÉTION DE PROSTASOMES ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.01.2018 KR 20180007024**
**16.01.2019 KR 20190005801**

(43) Date of publication of application:
**24.07.2019 Bulletin 2019/30**

(73) Proprietor: **Nambu University Industry-Cooperation Group Gwangju 62271 (KR)**

(72) Inventors:
• **PARK, Kwang Hyun**
**54823 JEONJU-SI (KR)**
• **OH, Yoon Wha**
**62258 GWANGJU (KR)**
• **KIM, Uh Hyun**
**54823 JEONJU-SI (KR)**
• **CHOI, Sun Eun**
**62322 GWANGJU (KR)**

(74) Representative: **Cabinet Becker et Associés 25, rue Louis le Grand 75002 Paris (FR)**

(56) References cited:
**EP-A1- 2 478 898    EP-A2- 0 179 442**

**US-B2- 8 263 059**

• **CA PALMERINI: "Increase of human spermatozoa intracellular Ca2+ concentration after fusion with prostasomes", CELL CALCIUM, vol. 25, no. 4, 25 May 2002 (2002-05-25), pages 291-296, XP055564291,**
• **PARK KWANG-HYUN ET AL: "Ca2+ signaling tools acquired from prostasomes are required for progesterone-induced sperm motility", SCIENCE SIGNALING, AMERICAN ASSOCIATION FOR THE ADVANCEMENT OF SCIENCE, US, vol. 4, no. 173, 1 January 2011 (2011-01-01), XP009158388, ISSN: 1937-9145, DOI: 10.1126/SCISIGNAL.2001595**
• **REN D: "Calcium signaling in sperm : help from prostasomes", SCIENCE SIGNALING, vol. 4, no. 173, 17 May 2011 (2011-05-17), page pe27, XP009511628,**
• **RONQUIST G ET AL: "The prostasome: its secretion and function in man", BIOCHIMICA ET BIOPHYSICA ACTA. MR. REVIEWS ON BIOMEMBRANES, ELSEVIER, AMSTERDAM, NL, vol. 822, no. 2, 9 September 1985 (1985-09-09), pages 203-218, XP025787757, ISSN: 0304-4157, DOI: 10.1016/0304-4157(85)90008-5 [retrieved on 1985-09-09]**
• **M Aalberts: "Prostasomes: extracellular vesicles from the prostate in: Reproduction Volume 147 Issue 1 Year 2014", , 1 January 2014 (2014-01-01), pages 1-14, XP055564353, Retrieved from the Internet: URL:https://rep.bioscientifica.com/view/jo urnals/rep/147/1/R1.xml?legid=reprod;147/1 /R1&related-urls=yes [retrieved on 2019-03-04]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD**

The present disclosure relates to a composition for regulating prostasome secretion, comprising an intracellular calcium signaling second messenger or a regulator thereof.

**BACKGROUND**

Motility of sperm is determined by motility which is retained after being inherently produced from the testes. However, in reality, various molecules are required to secure chemotaxis and active motility in the oocyte. It has been reported that these various molecules are supplied by prostasomes, the exosome secreted by the prostate (Park, et al., Sci Signal. 4 (173): ra31 (2011)).

The prostasomes have been reported to have a size of a few hundred nanometers (average 150 nanometers), and hundreds of proteins have been identified through protein analysis studies. Previously, these results have facilitated diagnosis of various diseases, development of therapeutic techniques thereof, and prognosis thereof (Tavoosidana et al., Proc Natl Acad Sci U.S.A. 108(21):8809-14 (2011), Fabiani et al., Hum. Reprod. 9, 1485-1489 (1994); Arienti et al., Biol. Cell 91, 51-54 (1999)). Prostasomes are specifically bound to sperms in a mildly acidic condition similar to that of female vaginas, resulting in structural or functional changes that have an absolute impact on fertility (Ronquist and Brody, Biochim. Biophys. Acta 822, 203-218 (1985), Arienti et al., Membr. Biol., 155, 89-94 (1997), and Publicover et al., Nat. Cell Biol., 9, 235-242 (2007); Burden et al., Hum. Reprod. Update 12, 283-292 (2006)).

Meanwhile, attempts have been conducted to develop techniques for application to industrial fields by identifying intracellular signaling mechanisms such as regulation of cholesterol of cell membranes (Llorente *et al.,* 2007) in the technology of activation and regulation of prostasome secretion. However, there has been no development of techniques for promoting and inhibiting pregnancy through increasing and decreasing the secretion of prostasomes by regulating the calcium signaling mechanisms inside and outside cells.

Thus, the present disclosure provides a method of regulating the prostasome secretion of based on the intracellular calcium signaling mechanisms in promoting and inhibiting the secretion of prostasomes, ultimately providing a method of improving pregnancy potential or achieving the purpose of contraception

**SUMMARY**

Thus, the present inventors have completed the present disclosure by confirming that it is possible to promote and inhibit the prostasome secretion based on the intracellular calcium signaling mechanism, ultimately confirming that it can be used to improve the possibility of pregnancy or achieve the purpose of contraception.

Therefore, the object of the present disclosure is to provide a composition for regulating prostasome secretion, comprising an intracellular calcium signaling second messenger or a regulator thereof.

Further, the object of the present disclosure is to provide techniques for improving or reducing sperm motility by activating or regulating the prostasome secretion by using techniques that regulate the intracellular calcium signaling mechanism.

In order to achieve the above objects, the present disclosure provides a composition for regulating prostasome secretion, comprising an intracellular calcium signaling second messenger or a regulator thereof.

Further, the present disclosure provides a method for *in vitro* regulating prostasome secretion, comprising the step of treating a prostasome secretion-regulating composition according to the present disclosure to prostate cells.

Further, the present disclosure provides a method for *in vitro* improving sperm motility, comprising the step of treating the composition to prostate cells.

Further, the present disclosure provides a method for screening a prostasome secretion enhancer or a sperm motility enhancer, the method comprising the steps of *in vitro* treating a candidate substance to prostate cells; measuring the calcium concentration in the prostate cells; and determining that the candidate substance is a prostasome secretion enhancer or a sperm motility enhancer when the calcium concentration in the prostate cells is higher than that before the candidate substance treatment as a result of the measurement of the calcium concentration.

The techniques to promote and regulate the prostasome secretion according to the present disclosure is mainly characterized by providing techniques for regulating exosome secretion based on the signaling mechanism in prostate cells. In particular, since it can directly regulate the secretion of prostasome that imparts motility to the sperm, it can be used for the verification of sperm motility in the diagnosis or treatment of infertility and can be used for the improvement of fertility and for contraception.

EP2478898 disclose the use of cyclic ADP-ribose for increasing sperm motility. Palmerini (1999), Cell Calcium, 25, 291-296, discloses an increase of intracellular calcium concentration in spermatozoa after fusion with prostasomes, thereby influencing sperm functions.

The foregoing summary is illustrative only and is not intended to be in any way limiting. In addition to the illustrative aspects, embodiments, and features described above, further aspects, embodiments, and features will become apparent by reference to the drawings and the following detailed description.

## BRIEF DESCRIPTION OF THE DRAWINGS

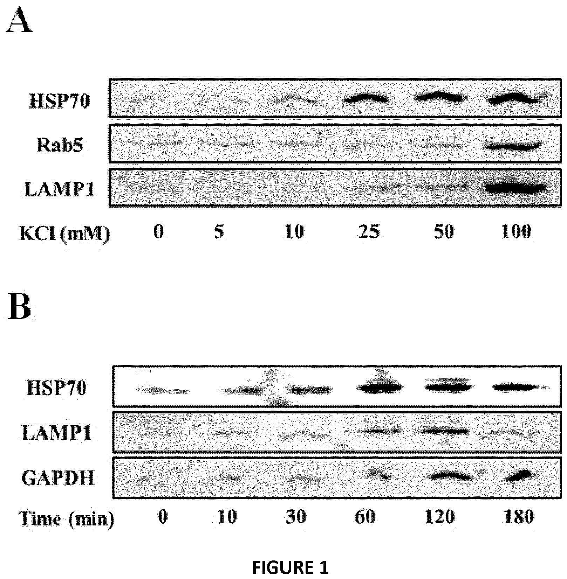

FIG. 1 illustrates Western blotting results for the verification of prostasome secretion increase by cell membrane depolarization of prostate cell lines, indicating the results of (A) concentration-dependent or (B) time-dependent prostasome secretion increase of representative marker molecules including HSP70, Rab5 and LAMP1, by treating with KCl;

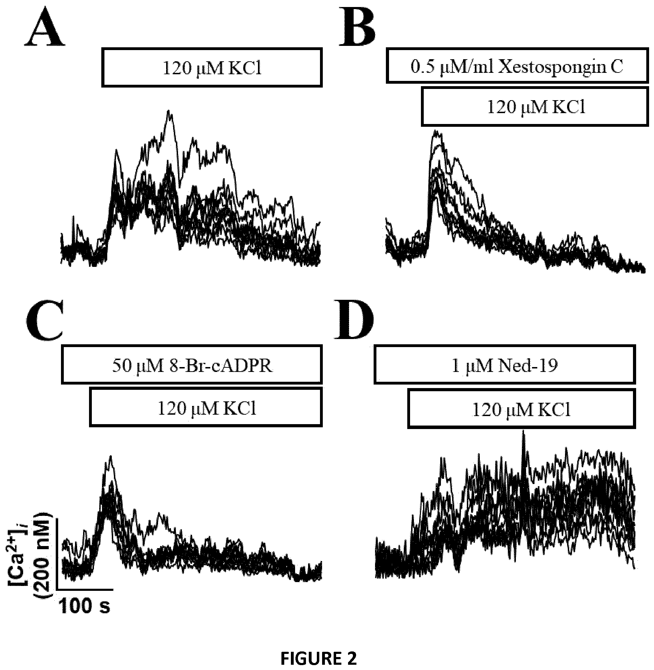

FIG. 2 illustrates changes in calcium signals related to prostasome secretion by cell membrane depolarization of prostate cell lines by pretreatment with regulators for the intracellular calcium signaling second messenger, indicating change in calcium signal controlling induced by (A) calcium signal induced by KCl treatment, (B) pretreatment with Xestospongin C (IP3 receptor antagonist), (C) pretreatment with 8-Br-cADPR (cyclic ADPR antagonist), (D) KCl treatment after pretreatment with Ned-19 (nicotinic acid adenine dinucleotide phosphate antagonist);

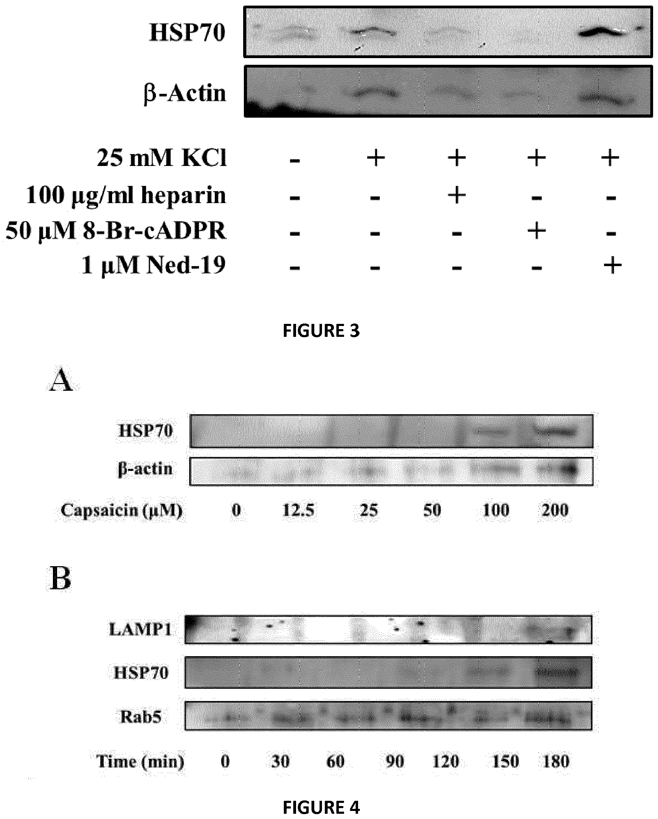

FIG. 3 illustrates Western blotting results for regulating action by intracellular calcium mobilization and alteration of regulators for secondary messengers by cell membrane depolarization of prostate cell lines (heparin: IP3 receptor antagonist, 8-Br-cADPR: cyclic ADPR antagonist, and Ned-19: nicotinic acid adenine dinucleotide phosphate antagonist);

FIG. 4 illustrates Western blotting results for verification of prostasome secretion by capsaicin and the like of prostate cell lines, indicating the results of (A) concentration-dependent or (B) time-dependent exosome secretion increase by treating capsaicin of HSP70, β-actin and LAMP1, representative marker molecules of exosomes containing prostasomes;

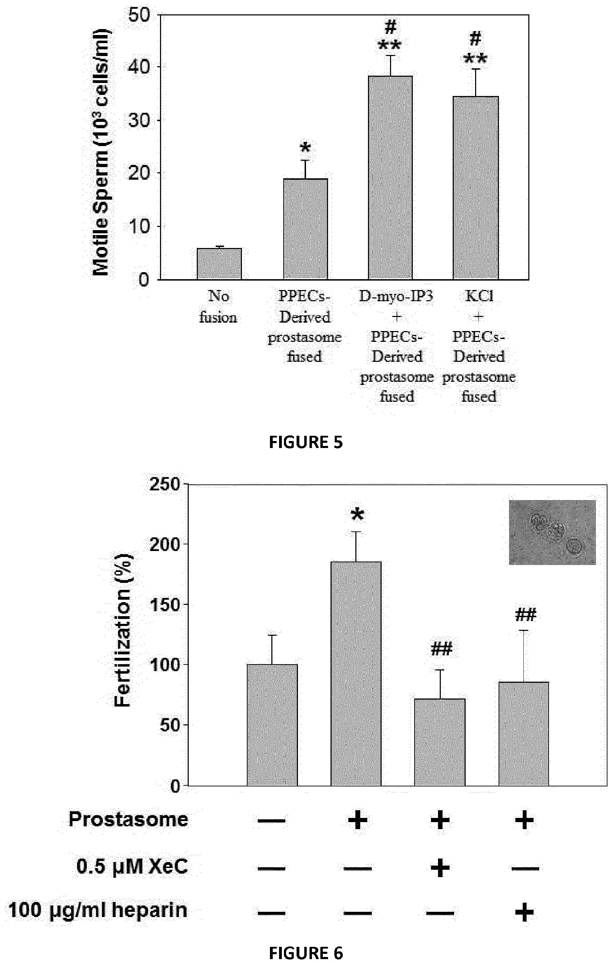

FIG. 5 illustrates the result of sperm motility test by the reaction with sperm and the prostasome secretion increase by treating the regulator of the intracellular calcium signaling second messenger or cell membrane depolarization (D-myo-IP3: D-myo-Inositol 1,4,5-triphosphate, KCl: potassiun phosphate; * $P < 0.05$, ** $P < 0.001$ vs. no fusion group; #$P < 0.05$ vs. PPECs-derived prostasome fused group);

FIG. 6 illustrates the results of controlling fertility by the reaction with sperm and the prostasome secretion increase by treating the regulator of the intracellular calcium signaling second messenger (XeC: Xestospongin C, * $P < 0.001$ vs. no prostasome incubated group; # $P < 0.05$ vs. prostasome incubated group), and the inset shows the fertilized egg in which the magnification is x40.; and

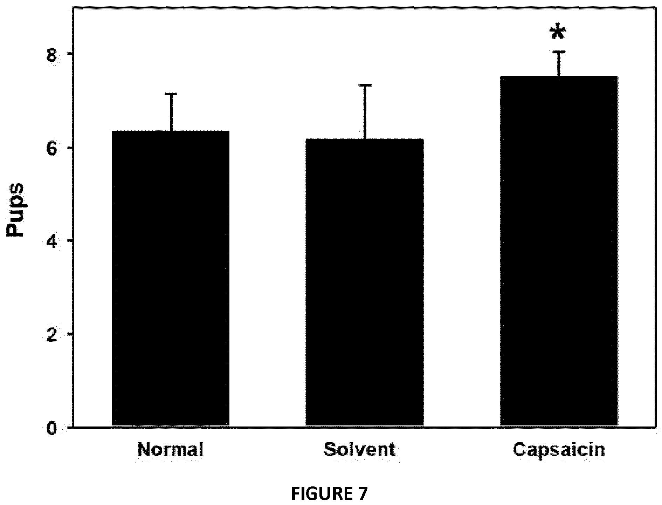

FIG. 7 illustrates the result of the increase in pups of mice by administration of capsaicin, which promotes the prostasome secretion (Normal: untreated group, Solvent: DMSO-PBS administrated group, Capsaicin: 50 mg/kg capsaicin administrated group) in which the results are expressed as mean $\pm$ standard derivation (SD) ($P < 0.05$ vs. Normal group).

## DETAILED DESCRIPTION

Hereinafter, the present disclosure is described in detail.

The present disclosure relates to a composition for regulating prostasome secretion, comprising an intracellular calcium signaling second messenger or a regulator thereof.

As used herein, the term "prostasome" refers to vesicles with a diameter of tens to hundreds of nanometers that are secreted by prostate cells, and they specifically binds to spermatozoa in mildly acidic environment such as vagina. The prostasomes can be isolated from normal prostate cells or malignant prostate cells, and the malignant prostate cell may be, but is not limited to, DU-145, LnCaP or PC-3. In one embodiment of the present disclosure, the prostasomes were isolated from DU-145.

As used herein, the term "second messenger" refers to a substance involved in the signaling pathway of an organism and particularly a small water-soluble substance, which is produced for secondarily transmitting and amplifying signals into cells when cells receive external signals.

Calcium is a multifunctional ion that maintains intracellular physiological balance by participating in the activity and inhibition of various enzymes, and in the stable state, the intracellular calcium concentration is maintained at about $10^{-7}$ M.

In the present disclosure, the calcium signaling second messenger that regulates the intracellular calcium signal refers to a substance that causes a corresponding reaction in the cell by changing the calcium concentration inside and outside the cell. The second messenger may be, but is not limited to, cyclic ADP-ribose (cADPR) or inositol 1,4,5-

trisphosphate ($IP_3$).

IP3 fuses with the IP3 receptor to release calcium from the endoplasmic reticulum (ER), which is a cell organelle. Other second messengers, NAD and NADP, are metabolized into cyclic ADP-ribose (cADPR) and NAADP (nicotinic acid adenine dinucleotide phosphate), respectively, by NAD glycohydrolase (NADase). NAADP causes calcium release from lysosomes-like organelles, while cADPR causes calcium release from the endoplasmic reticulum (ER)/sarcoplasmic reticulum (SR) by calcium-induced calcium release (CICR) mechanism.

In one embodiment of the present disclosure, the intracellular calcium signaling mechanism is analyzed by pretreatment with the antagonist to each of the intracellular calcium signaling second messengers listed above. The results indicate that the calcium signaling pathway for prostasome secretion passes through the pathway via IP3 and cADPR, but not through the NAADP signal.

Further, the calcium signaling second messenger may be cyclic ADP-ribose, inositol 1,4,5-trisphosphate or derivatives thereof. In one embodiment of the present disclosure, D-myo-Inositol 1,4,5-triphosphate (D-myo-$IP_3$) is used as a regulator of intracellular calcium signaling second messenger to directly raise intracellular IP3 concentration, thereby increasing the intracellular calcium concentration and prostasome secretion from prostate cells.

Further, in one embodiment of the present disclosure, capsaicin is used as a substance that promotes prostasome secretion by regulating the calcium signaling mechanism inside and outside the prostate cells. The capsaicin is a substance that induces cell signaling via an ion channel such as TRPV1, which has been approved by the FDA for specific purposes.

In the present disclosure, the regulation of prostasome secretion may be the inhibition or promotion of secretion of prostasomes, which may be selected according to the purpose of using the composition for regulating prostasome secretion according to the present disclosure. For example, antagonists to intracellular calcium signaling second messengers may inhibit the prostasome secretion and inducers of intracellular calcium signaling second messengers may increase the prostasome secretion.

The composition according to the present disclosure can be administered orally or parenterally at the time of clinical administration and can be formulated into various general pharmaceutical formulations.

In the case of formulation, a diluent or excipient such as a commonly used filler, an extender, a binder, a wetting agent, a disintegrant, or a surfactant may be used. Solid formulations for oral administration may be formulated by mixing one or more excipients such as starch, calcium carbonate, sucrose, lactose or gelatin to one or more compositions of the present disclosure. In addition to simple excipients, a lubricant such as magnesium stearate or talc may be used. Liquid preparations for oral administration include a suspension, a solution, an emulsion or a syrup. Various excipients such as a wetting agent, a sweetener, a fragrance or a preservative may be included in addition to commonly used simple diluents such as water and liquid paraffin. Formulations for parenteral administration include a sterile aqueous solution, a non-aqueous solution, a suspension, an emulsion, a freeze-dried preparation or a suppository. As the non-aqueous solution or suspension, propylene glycol, polyethylene glycol, vegetable oil such as olive oil, injectable ester such as ethyl oleate, and the like can be used. As a suppository base, witepsol, macrogol, tween 61, cacao butter, laurinum, glycerol or gelatin may be used.

Further, the dose of the composition according to the present disclosure may be varied depending on the patient's age, body weight, sex, dosage form, health condition, and the severity of the disease. The composition may be administered in an amount of typically about 0.1 mg/day to about 1000 mg/day, preferably 1 mg/day to 500 mg/day, and most preferably 0.7 mg/day to 3.5 mg/day, based on adult patients having the weight of 70 kg. The administration may be carried out in an appropriate interval, e.g., in a single dose or in divided doses per day, according to the doctor's or pharmacist's instruction.

Further, the present disclosure provides a method for regulating prostasome secretion, the method comprising the step of treating a prostate cell with the composition for *in vitro* regulating the prostasome secretion according to the present disclosure.

Further, the present disclosure provides a method for improving sperm motility, the method comprising the step of treating a prostate cell with the composition for *in vitro* regulating the prostasome secretion according to the present disclosure.

In one embodiment of the present disclosure, D-myo-Inositol 1,4,5-triphosphate (D-myo-$IP_3$) is used as a composition for regulating prostasome secretion according to the present disclosure. Further, cells are treated with D-myo-$IP_3$ which may directly increase the concentration of IP3, an intracellular calcium signaling second messenger to effectively induce the prostasome release of prostate cells, resulting in a doubling of sperm motility.

In the present disclosure provides a method for improving sperm motility, the method further comprising the step of fusing prostasomes obtained from the prostate cells with isolated sperms.

Further, the present disclosure provides a method for screening a prostasome secretion enhancer, the method comprising the steps of: *in vitro* treating a candidate substance to prostate cells; measuring the calcium concentration in the prostate cells; and determining that the candidate substance is a prostasome secretion enhancer when the calcium concentration in the prostate cells is higher than that before the candidate substance treatment as a result of the measurement of the calcium concentration.

Further, the present disclosure provides a method for screening a sperm motility enhancer, the method comprising the steps of: *in vitro* treating a candidate substance to prostate cells; measuring the calcium concentration in the prostate cells; and determining that the candidate substance is a sperm motility enhancer when the calcium concentration in the prostate cells is higher than that before the candidate substance treatment as a result of the measurement of the calcium concentration.

Hereinafter, the present disclosure is described in detail with experimental examples and embodiments. However, the following experimental examples and embodiments are merely illustrative of the present disclosure, and the content of the present disclosure is not limited to the following experimental examples and embodiments.

## Experimental Example 1. Isolation of prostasomes

Prostasomes were isolated from human prostate cancer cell line culture medium. Methods described in Palmerini *et al.* (Palmerini et al., Fertil. Steril. 80, 1181-1184 (2003)) were modified and used for the separation of prostasomes.

Specifically, the prostate cancer cell line DU-145 were cultured in DMEM (GIBCO/Invitrogen, USA) supplemented with 10% exosome-free FBS (GIBCO/Invitrogen, USA) and 1% antibiotics-antimycotic (GIBCO/Invitrogen, USA) under the conditions of 37°C and 5% $CO_2$. The cell culture was collected and centrifuged at 3,000 rpm for 30 minutes to separate the supernatant. The supernatant was centrifuged at 15,000 xg for 20 minutes using a Beckman's high-speed centrifuge, and the supernatant was separated again. The supernatant was centrifuged at 105,000 xg for 2 hours using a high-speed centrifuge to obtain a precipitate. The collected precipitate was resuspended in the same buffer. The first eluted fraction was collected using sephadex G-200 (Sigma, USA), centrifuged again, and then suspended for use.

The prostasomes of the mouse were obtained by incising the mouse abdominal cavity and obtaining the prostate under the bladder through microsurgery (Drost et al, 2016, Nat Protoc. 11(2): 347- 358). Later, the effluent from the prostate gland was collected from the prostate and diluted using BWW (Bigger, Whitten, and Whittingham) medium [10 mM Hepes, 20 mM sodium lactate, 5 mM glucose, 0.25 mM sodium pyruvate, penicillin G (80 mg/L), streptomycin sulfate (50 mg/L), 95 mM NaCl, 4.8 mM KCl, 1.3 mM $CaCl_2$, 1.2 mM $KH_2PO_4$, and 1.2 mM $MgSO_4$ in 25 mM $NaHCO_3$ buffer, pH 7.4]). The supernatant was used to collect prostasomes by the same method as described above.

## Experimental Example 2. Isolation of sperms

To collect mouse sperms which are not fused with prostasomes, the tail epididymis of a male mouse was surgically removed, and blood and adipose tissue were removed using ophthalmic scissors. The epididymis was punctured with a 30 gauge sterilized needle and pinched by tweezers to release a large number of sperms. The sperm suspension was immediately diluted 20 times with BWW (Bigger, Whitten, and Whittingham) medium [10 mM Hepes, 20 mM sodium lactate, 5 mM glucose, 0.25 mM sodium pyruvate, penicillin G (80 mg/L), streptomycin sulfate (50 mg/L), 95 mM NaCl, 4.8 mM KCl, 1.3 mM $CaCl_2$, 1.2 mM $KH_2PO_4$, and 1.2 mM $MgSO_4$ in 25 mM $NaHCO_3$ buffer, pH 7.4]) containing 1% bovine serum albumin (BSA) preheated to 37°C and centrifuged at 900 xg for 5 minutes at room temperature, and the precipitate was washed twice with the same buffer for use.

## Experimental Example 3. Western Blotting

20 µg of protein was inactivated in 5 × sample buffer at 100°C for 5 minutes and then electrophoresed on a 10% SDS polyacrylamide gel. The separated proteins were transferred to a PVDF membrane and blocked in 2% BSA for 90 minutes. The respective HSP70, β-actin, Rab5 and LAMP1 antibodies were placed overnight at 4°C in a ratio of 1 : 1,000 and then washed three times for 10 minutes with tris-buffered saline Tween-20 (TBST) buffer. The secondary antibodies were diluted at a ratio of 1 : 1,000 and allowed to adhere at room temperature for 2 hours. The membranes washed three times for 15 minutes with TBST buffer were treated with ECL (Bio-Rad, USA), and the amount of the protein expression was measured with LAS-500 (GE Healthcare Life Science, USA).

## Embodiment 1. Effects of intracellular calcium signaling regulation on prostasome production and secretion

### 1-1. Analysis of prostasome-related protein expression by KCl treatment

DU-145, prostate cancer cell line, was treated with KCl, which induces cell membrane depolarization. Western blotting was performed to identify changes in prostasome-related protein expression. Specifically, the cells were cultured with KCl at various concentrations (0, 5, 10, 25, 50 and 100 mM) for 1 hour, and prostasomes were prepared from the culture supernatant by the method described in Experimental Example 1 and subjected to Western blotting. Further, cells were cultured with 25 mM KCl for various times (0, 10, 30, 60, 120 and 180 minutes) and subjected to Western blotting. The results are illustrated in FIG. 1.

As illustrated in FIG. 1A, it was confirmed that by KCl treatment, protein expression of HSP70, Rab5 and LAMP1, which are typical exosomal markers, was increased in a concentration-dependent manner in the human prostate cancer cell line DU-145. Further, as illustrated in FIG. 1B, it was confirmed that protein expression of HSP70 and LAMP1 was also increased in a time-dependent manner. The above results confirm that the treatment of KCl induces the membrane depolarization, resulting in the release of prostasomes and that the increase degree of prostasome release can be determined from the increase of the expression level of the exosomal marker.

**1-2. Analysis of intracellular calcium signaling by treating with regulator of calcium signaling second messenger**

DU-145, human prostate cancer cell line, was pretreated with a regulator of intracellular calcium ($Ca^{2+}$) signaling second messenger, and the intracellular calcium signaling pattern thereof was analyzed. The concentration of intracellular calcium was measured by the following method: A cell suspension pretreated with a fluo-3 (molecular probe, USA) was titrated in a confocal dish (SPL, Seoul, Korea) coated with poly-L-lysine (Sigma-Aldrich, USA) and reacted for 20 minutes in a $CO_2$ incubator to attach the cells. Then, the intracellular calcium amount was measured by a confocal microscopy system of Nikon Co. using the method of Tsien *et al.* (Tsien et al., Nature 295, 68-71 (1982)), and the results are illustrated in FIG. 2.

As illustrated in FIG. 2A, it was confirmed that the cells were treated with 120 $\mu$M KCl, inducing that intracellular calcium ($[Ca^{2+}]_i$) was rapidly increased and calcium was continuously increased. In this model, it was confirmed that the continuous increase of $[Ca^{2+}]_i$ by KCl treatment was suppressed when pretreated with competitive $IP_3$ receptor antagonist, Xestospongin C (FIG. 2B). Similarly, it was confirmed that the continuous increase of $[Ca^{2+}]_i$ was suppressed even when pretreated with 8-br-cADPR, which is an antagonist of cADPR (FIG. 2C). On the other hand, it was confirmed that the continuous increase of $[Ca^{2+}]_i$ was not suppressed when pretreated with Ned-19, which is an antagonist of NAADP (FIG. 2D). The above results show that KCl increases intracellular calcium concentration and increases prostasome production and secretion, and the calcium signaling mechanism for prostasome secretion passes through IP3 and cADPR but does not pass through NAADP signal.

**1-3. Analysis of prostasome-related protein expression by treating with regulator of calcium signaling second messenger**

Furthermore, DU-145, human prostate cancer cell line was pretreated with a regulator of intracellular calcium ($Ca^{2+}$) signaling second messenger to analyze the change in the prostasome-related protein expression. Specifically, DU-145 cells were treated with various antagonists (regulator of intracellular calcium signaling second messenger) such as 100 $\mu$g/ml heparin (selective IP3 receptor inhibitor), 50 $\mu$M 8-Br-cADPR (antagonist of cADPR) and 1 $\mu$M NED-19 (antagonist of NAADP) together with 25 mM KCl. Prostasomes were prepared from the culture supernatant by the method described in Experimental Example 1, and Western blotting was performed. All antagonists were preincubated for 30 minutes before KCl treatment, and the cells were cultured for an additional 1 hour after KCl treatment. The results are illustrated in FIG. 3.

As illustrated in FIG. 3, it was confirmed that the HSP70 expression was increased in the group treated with KCl alone without an antagonist, but the HSP70 expression was suppressed in the group treated with an antagonist and KCl. On the other hand, it was confirmed that the group treated with Ned-19 did not show the inhibitory effect of HSP70 expression. These results indicate that HSP70, which is increased in expression due to KCl, is reduced when it is treated with an antagonist for IP3 and cADPR (inhibitor of calcium signaling second messenger). Furthermore, HSP70 is a protein marker in the prostasome derived from the prostate cancer cell line, and thus the fact indicates that prostasome secretion is reduced upon treatment of the regulator of calcium signaling second messenger.

**1-4. Analysis of prostasome-related protein expression by treating with capsaicin**

Capsaicin (Sigma Aldrich, MO, USA), which induces intracellular calcium ($Ca^{2+}$) signaling, was used to confirm the change in prostasome-related protein expression according to capsaicin treatment in DU-145, human prostate cancer cell line by Western blot. Specifically, the cells were cultured with capsaicin at various concentrations (0, 12.5, 25, 50, 100 and 200 $\mu$M) for 3 hours, and prostasomes were prepared from the culture supernatant by the method described in Example 2 and subjected to Western blotting. The cells were also cultured with 50 $\mu$M capsaicin for various times (0, 10, 30, 60, 91, 120 and 180 minutes) and subjected to Western blotting. The results are illustrated in FIG. 4.

As illustrated in FIG. 4A, it was confirmed that the protein expression of HSP70, a typical exosomal marker, was increased in a concentration-dependent manner in human prostate cancer cell line DU-145 by capsaicin treatment. Further, as illustrated in FIG. 4B, it was confirmed that the protein expression of HSP70, LAMP1 and Rab5 was also increased in a time-dependent manner. The above results confirm that the treatment of capsaicin induces the membrane depolarization, resulting in the release of prostasomes and that the increase degree of prostasome release can be determined from the increase of the expression level of the exosomal marker.

**Embodiment 2. Sperm motility test by prostasome**

The prostasomes and sperms were obtained from the mouse's normal prostate cell lines (PPECs) in the same manner as in Experimental Examples 1 and 2. It was examined how the fusion of sperms with prostasomes affects sperm motility. In addition to the control group not fused with prostasomes, the following three experimental groups were divided: sperms fused with prostasomes (PPECs-Derived prostasome fused), sperms fused with prostasomes derived from mouse's normal prostate cell lines pretreated with D-myo-Inositol 1,4,5-triphosphate (D-myo-IP$_3$) (D-myo-IP$_3$ + PPECs-Derived prostasome fused), and sperms fused with to prostasomes derived from mouse's normal prostate cell lines pretreated with KCl (KCl + PPECs-Derived prostasome fused).

Specifically, the isolated sperms were diluted with a weak alkaline buffer with 0.32 M sucrose or binding buffer 150 mM NaCl, 1 mM CaCl$_2$, 1 mM MgCl$_2$, 5 mM glucose with 2 mM Hepes (pH 8.0)/20 mM MES (pH 5.0, binding buffer). Then, the prostasomes and sperm proteins were mixed at a ratio of 2 : 1 and reacted at 37°C for 15 minutes. The reaction product was centrifuged at 600 xg for 10 minutes. Then, the precipitate was collected and resuspended in BWW buffer for test. The sperm motility rate was measured using a computer assisted sperm analysis (CASA) device (IVOS, Hamilton Thorne Biosciences, USA). The sperm suspension was titrated on a 20-micron depth sperm analysis slide (2X-CEL, Hamilton Thorne Biosciences, USA), and auto-photographing and analysis were performed through a 4x magnification lens. The results are illustrated in FIG. 5.

As illustrated in FIG. 5, the sperm motility of the sperm fused with prostasomes was increased within the error range as compared with the sperm that was not fused with the prostasome, but such pattern was not significant. It was confirmed that the sperm motility rate was doubled by treating the sperm with D-myo-IP$_3$ which can directly elevate the concentration of IP3, which is an intracellular calcium signaling second messenger. These results were confirmed by the results of measuring velocity of curvilinear (VCL), velocity of straight-line (VSL), velocity of average path (VAP) and hyperactivation. These results demonstrate that D-myo-IP$_3$, a representative IP3 inducer, increases intracellular calcium influx, thereby effectively causing prostasome release of prostate cells. Particularly, the D-myo-IP$_3$ is generally known to be cell-impermeable. However, from these results, the present inventors have found the IP3 cell permeability, intracellular calcium signal induction thereof, and the increase of prostasome release by increasing intracellular calcium signal.

**Embodiment 3. *In vitro* fertilization test**

In order to perform the *in vitro* fertilization test, the method of Ren *et al.* (Ren et al., Nature 413, 603-609 (2001)) was modified and used. A 10 to 12-week-old female mouse was injected with pregnant mare serum gonadotropin (PMSG) (5 IU; Sigma-Aldrich). After 48 hours, the mouse was injected with human chorionic gonadotropin (hCG) (5 IU; Sigma-Aldrich). After 14 hours of the last injection, uterine tubes were removed and perfused with IVF medium (Medicult, USA), and flown oocytes were collected using a low magnification microscope.

The collected oocytes were resuspended in IVF medium and were cultured with prostasomes which were obtained in the presence or absence of the regulator of the intracellular calcium signaling second messenger, causing fertilization reaction. The heated mineral oil was overlaid on top of the medium to block the outside air. After 36 hours of the culture, the total number of oocytes contained in the culture medium was counted under a microscope, and cells above the two-cell stage were counted. The fertilization rate was calculated using the following formula. The case that the sperms that had undergone the same fusion process without prostasome treatment were cultured with about 15 oocytes was referred to as 100%. The results are illustrated in FIG. 6.

$$\text{Fertilization rate (\%)} = \text{Number of fertilized oocytes above the two-cell stage}$$

$$(\text{counts}) \div \text{Total number of oocytes (counts)} \times 100$$

As illustrated in FIG. 6, it was confirmed that when the sperms fused with prostasomes derived from mouse's normal prostate cells were reacted with oocytes, the fertility thereof was increased about twice. On the other hand, it was confirmed that the fertility of sperms fused with prostasomes obtained by pretreatment of Xestospongin C (XeC) and heparin, which is a selective IP3 receptor inhibitor, was reduced to be similar to that of the negative control. These results indicate that the secretion of prostasome passes through the IP3 receptor in the calcium signaling pathway, and the fertility can be effectively controlled by regulating prostasome secretion.

**Embodiment 4.** *In vivo* **fertilization test in mice**

The number of pups was measured in the mouse breeding process to confirm the increase of reproductive ability due to the promotion of the prostasome secretion (Nambu University's animal test approval number #201604). 7-week-old male C57BL/6 mice were randomly assigned into three groups and adapted to the breeding conditions. The groups were divided into the normal untreated group, the solvent-treated group, and the capsaicin-treated group. Capsaicin was prepared at a high concentration (100-fold) in DMSO, then diluted in phosphate buffered saline (PBS, pH 7.2), and used as an injection. In the solvent-treated group, PBS (200 $\mu$l) diluted with the same volume of DMSO was administered. Twice daily capsaicin (50 mg/kg) and placebo were intraperitoneally injected into each experimental group for 2 weeks. Then, each mouse was caged with a female c57BL/6 mouse adapted for the same breeding condition, mating them at a ratio of 1 : 1 for 48 hours. The mating started at 6 pm, and the mating success was evaluated by confirming that the plaque was formed at the vaginal entrance at 9:00 am, but it was maintained for 48 hours considering the possibility of premature dropping. Then, only the female was separated from each cage, and the separate breeding was performed for each group. After the gestation period (about 21 days), the number of pups was counted. As a result, student t-test was conducted to evaluate the significance thereof. The results are illustrated in FIG. 7.

As illustrated in FIG. 7, it was confirmed that the number of pups was increased in the capsaicin-treated group. From the above results, it was confirmed that the reproductive ability of mouse was increased by promoting prostasome secretion due to the capsaicin treatment.

As confirmed in the above Embodiments, the present inventors have confirmed that the regulator of the intracellular calcium signaling second messenger is used to regulate the calcium signaling pathway inside and outside the cells, thereby controlling the prostasome production and secretion of the prostate cells to effectively control pregnancy. Therefore, the techniques to promote and regulate prostasome secretion according to the present disclosure can be used for the improvement of fertility and for contraception.

From the foregoing, it will be appreciated that various embodiments of the present disclosure have been described herein for purposes of illustration.

**Claims**

1. An *in vitro* method for regulating prostasome secretion, the method comprising the step of treating a prostate cell with an intracellular calcium signaling second messenger or a regulator thereof, wherein the second messenger is one selected from the group consisting of cyclic ADP-ribose, inositol 1,4,5-trisphosphate and derivates thereof and wherein the regulator of intracellular calcium signaling second messenger is D-myo-Inositol 1,4,5-triphosphate (D-myo-IP3) or capsaicin..

2. An *in vitro* method for improving sperm motility, the method comprising the step of treating a prostate cell with an intracellular calcium signaling second messenger or a regulator thereof, wherein the second messenger is one selected from the group consisting of cyclic ADP-ribose, inositol 1,4,5-trisphosphate and derivates thereof and wherein the regulator of intracellular calcium signaling second messenger is D-myo-Inositol 1,4,5-triphosphate (D-myo-IP3) or capsaicin.

3. The *in vitro* method according to claim 2, the method comprising the step of fusing prostasomes obtained from the prostate cell with isolated sperms.

**Patentansprüche**

1. In-vitro-Verfahren zur Regulierung der Prostasom-Sekretion, wobei das Verfahren den Schritt der Behandlung einer Prostatazelle mit einem intrazellulären Calcium-Signal-Second-Messenger oder einem Regulator davon umfasst, wobei der Second-Messenger einer ist, der aus der Gruppe bestehend aus cyclischer ADP-Ribose, Inosit-1,4,5-trisphosphat und Derivaten davon ausgewählt ist, und wobei der Regulator des intrazellulären Calcium-Signal-Second-Messengers D-Myo-Inosit-1,4,5-Triphosphat (D-Myo-IP3) oder Capsaicin ist.

2. In-vitro-Verfahren zur Verbesserung der Spermienmotilität, wobei das Verfahren den Schritt der Behandlung einer Prostatazelle mit einem intrazellulären Calcium-Signal-Second-Messenger oder einem Regulator davon umfasst, wobei der Second-Messenger einer ist, der aus der Gruppe bestehend aus cyclischer ADP-Ribose, Inosit-1,4,5-trisphosphat und Derivaten davon ausgewählt ist, und wobei der Regulator des intrazellulären Calcium-Signal-Second-Messengers D-Myo-Inosit-1,4,5-Triphosphat (D-Myo-IP3) oder Capsaicin ist.

**3.** In-vitro-Verfahren nach Anspruch 2, wobei das Verfahren den Schritt des Verschmelzens von Prostasomen, die aus der Prostatazelle erhalten wurden, mit isolierten Spermien umfasst.

**Revendications**

**1.** Méthode *in vitro* pour réguler la sécrétion de prostasomes, la méthode comprenant l'étape de traitement d'une cellule de prostate avec un second messager de signalisation du calcium intracellulaire ou un régulateur de celui-ci, dans laquelle le second messager est l'un choisi dans le groupe constitué par l'ADP-ribose cyclique, le 1,4,5-trisphosphate d'inositol et leurs dérivés, et dans laquelle le régulateur du second messager de signalisation du calcium intracellulaire est le 1,4,5-triphosphate de D-myo-inositol (D-myo-IP3) ou la capsaïcine.

**2.** Méthode *in vitro* pour améliorer la motilité des spermatozoïdes, la méthode comprenant l'étape de traitement d'une cellule de prostate avec un second messager de signalisation du calcium intracellulaire ou un régulateur de celui-ci, dans laquelle le second messager est l'un choisi dans le groupe constitué par l'ADP-ribose cyclique, le 1,4,5-trisphosphate d'inositol et leurs dérivés, et dans laquelle le régulateur du second messager de signalisation du calcium intracellulaire est le 1,4,5-triphosphate de D-myo-inositol (D-myo-IP3) ou la capsaïcine.

**3.** Méthode *in vitro* selon la revendication 2, la méthode comprenant l'étape de fusion des prostasomes obtenus à partir de la cellule de prostate avec des spermatozoïdes isolés.

A

B

FIGURE 1

**FIGURE 2**

| | | | | | |
|---|---|---|---|---|---|
| HSP70 | | | | | |
| β-Actin | | | | | |
| 25 mM KCl | − | + | + | + | + |
| 100 µg/ml heparin | − | − | + | − | − |
| 50 µM 8-Br-cADPR | − | − | − | + | − |
| 1 µM Ned-19 | − | − | − | − | + |

**FIGURE 3**

A

| | | | | | | |
|---|---|---|---|---|---|---|
| HSP70 | | | | | | |
| β-actin | | | | | | |
| Capsaicin (µM) | 0 | 12.5 | 25 | 50 | 100 | 200 |

B

| | | | | | | | |
|---|---|---|---|---|---|---|---|
| LAMP1 | | | | | | | |
| HSP70 | | | | | | | |
| Rab5 | | | | | | | |
| Time (min) | 0 | 30 | 60 | 90 | 120 | 150 | 180 |

**FIGURE 4**

**FIGURE 5**

| Prostasome | — | + | + | + |
| 0.5 µM XeC | — | — | + | — |
| 100 µg/ml heparin | — | — | — | + |

**FIGURE 6**

FIGURE 7

**EP 3 514 538 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2478898 A

**Non-patent literature cited in the description**

- **PARK et al.** *Sci Signal.,* 2011, vol. 4 (173), ra31
- **TAVOOSIDANA et al.** *Proc Natl Acad Sci U.S.A.,* 2011, vol. 108 (21), 8809-14
- **FABIANI et al.** *Hum. Reprod.,* 1994, vol. 9, 1485-1489
- **ARIENTI et al.** *Biol. Cell,* 1999, vol. 91, 51-54
- **RONQUIST ; BRODY.** *Biochim. Biophys. Acta,* 1985, vol. 822, 203-218
- **ARIENTI et al.** *Membr. Biol.,* 1997, vol. 155, 89-94
- **PUBLICOVER et al.** *Nat. Cell Biol.,* 2007, vol. 9, 235-242
- **BURDEN et al.** *Hum. Reprod. Update,* 2006, vol. 12, 283-292
- **PALMERINI.** *Cell Calcium,* 1999, vol. 25, 291-296
- **PALMERINI et al.** *Fertil. Steril.,* 2003, vol. 80, 1181-1184
- **DROST et al.** *Nat Protoc.,* 2016, vol. 11 (2), 347-358
- **TSIEN et al.** *Nature,* 1982, vol. 295, 68-71
- **REN et al.** *Nature,* 2001, vol. 413, 603-609